# EUROPEAN PATENT SPECIFICATION

(11) **EP 3 930 564 B1**
(45) Date of publication and mention of the grant of the patent: **05.07.2023**
(21) Application number: 20706264.7
(22) Date of filing: 21.02.2020
(51) Int. Cl.: A61B 5/00, A61B 5/107

(54) **METHOD FOR OPERATING A 3D BODY SCANNER**
VERFAHREN ZUM BETRIEB EINES 3D-KÖRPERSCANNERS
PROCÉDÉ DE FONCTIONNEMENT D'UN SCANNER CORPOREL 3D

(30) Priority: 27.02.2019 AT 600512019
(43) Date of publication of application: 05.01.2022
(73) Proprietor: Naked Labs Austria GmbH, 1070 Wien (AT)
(72) Inventor: SCHULTES, Gerhard, 1220 Wien (AT); FARAHBAKHSHIAN, Farhad, Redwood City, California 94063 (US); KREUZGRUBER, Peter, 1070 Wien (AT)
(74) Representative: Canzler & Bergmeier Patentanwälte Partnerschaft mbB
(86) International application number: PCT/EP2020/054699
(87) International publication number: WO 2020/173842

(56) References cited:
- WO-A1-2016/179448
- WO-A1-2018/011334
- JP-A- 2007 243 634
- US-A1- 2007 172 112
- US-A1- 2011 054 310

## Description

The invention relates to a method for operating a 3D body scanner for generating a 3D body model with the following steps: measuring an installation geometry of the 3D body scanner, calculating an optimal scanning distance between a person and the 3D body scanner by a control unit and projecting an optical indicator for the person to be scanned at a floor in front of the 3D body scanner at the optimal scanning distance from the 3D body scanner by a position indicator projector.

Today accurate 3D human body models are demanded by various fields of applications as are:
- Fitness and body styling application,
- Medical applications,
- Cloth manufacturing industry,
- Cloth internet- and retail shops and/or
- Automotive industry.

To generate 3D body models 3D body scanners are used. For high performance scans the human body must be placed as close as possible to the scanner but not so close that it is exceeding its Field of View (FOV).

The field of the disclosed invention deals with automatic creating a visible indication in optimum distance on the floor in front of the scanner where the user should step on for scanning. The distance is automatic adapted depending on how the scanner is installed in a room.

If the user is too close to the scanner, parts of its body will be cut-off at the top, bottom and/or left or right. If a user is too far it will typically result in a low-resolution scan. State of the art 3D body scanners are well defined installed by standing on a flat floor.

Home scanners are scanning while standing on a turntable mechanically separated from a scanner mast. The turntable is placed manually on the floor in front of the scanner. The concept works well because installation is very easy and there is only a low chance to set an improper distance.

A 3D body scanner with a mast and a turntable is discloses in WO 2018/011334 A1. The mast may project an optical marker on a floor that indicates a user where the turntable has to be placed relative to the mast. Turntable-less 3D body scanners let the person by its own motion turn around at a roughly defined speed.

Common disadvantage of the solutions above is that they need a fix and inflexible construction determining the height and angle of view of the depth sensors installed in the 3D body scanners. This is leading to:
(i) Large scanner constructions which need to be placed on the floor or;
(ii) When considering small, more handy scanners, the need to be installed under fulfilling strict geometrical conditions, which should also not be changed uncontrollably at operation. The required skills and tools can hardly be demanded from an average user. Also changing the operating conditions can hardly be avoided in a normal living space.

The disadvantages will be solved by a method for operating a 3D body scanner and a 3D body scanner according to the independent claims.

The present invention is defined in independent claims 1 and 11. Further embodiments are defined in dependent claims.

It is proposed a method operating a 3D body scanner for generating a 3D body model.

At a first step an installation geometry of the 3D body scanner will be measured. Installation geometry means, for example, a position and/or orientation of the 3D body scanner according to a room and/or a floor.

At a further step, a control unit will calculate an optimal scanning distance between a person and the 3D body scanner. The distance must not be too great, otherwise the resolution of the scanner will be degraded. On the other hand, the person must not get too close to the scanner, otherwise the person cannot be fully captured. In general, the optimal scanning distance is as close as possible to the 3D body scanner. Therefore, the optimal scanning distance should be as short as possible. Furthermore, the optimal scanning distance indicates the optimal scanning position in front of the 3D body scanner. The optimal scanning position may be furthermore in the middle in front of the 3D body scanner.

At a further step, an optical indicator for the person to be scanned will be projected on a floor in front of the 3D body scanner at the optimal scanning distance from the 3D body scanner by a position indicator projector. The person to be scanned can move to the optical indicator so that the 3D body scanner can capture the person as well as possible. Therefore, the 3D body scanner can be arbitrarily placed in the room. For example, it does not matter, whether the 3D body scanner is placed on a high or a low furniture.

At a further advantageous step, for the installation geometry a height of the 3D body scanner above the floor in front of the 3D body scanner will be measured.

At a further advantageous step, for the installation geometry an angle of the 3D body scanner 104 according to a vertical axis will be measured.

At a further advantageous step, for calculating of the optimal scanning distance a field of view (FOV) of a depth sensor of the 3D body scanner 104 is taken into account.

At a further advantageous step, for calculating of the optimal scanning distance a field of view (FOV) of a color camera of the 3D body scanner 104 is taken into account. The color camera may be a RGB camera.

At a further advantageous step, the field of view (FOV) will be scanned by the depth sensor and/or by the color camera. At a further step, a depth map will be generated by the control unit on basis of the data of the depth sensor and/or of the color camera. The depth map may contain distance information for each point (according to the resolution of the depth sensor and/or the color camera) of the area of the field of view. The field of view may be 2-dimensional. The field of view may have a width and a height. The field of view further has a vertical and a horizontal expansion. For example, the body height of the person to be scanned can be determined from the depth map.

At a further advantageous step, the floor in front of the 3D body scanner on basis of the depth map will be identified. In general, the floor is a flat plane. This can be analyzed or recognized to identify the floor.

At a further advantageous step, a body height of the person to be scanned will be determined. For example, the control unit can determine the body height on basis of data from the depth sensor and/or the color camera. Furthermore, the body height can be determined on basis of the depth map. In addition or alternatively the body height can be assumed. For example, a body height of 1.80 meter, 1.90 meter or 2 meters can be assumed.

At a further advantageous step, the height of the 3D body scanner above the floor and/or the angle of the 3D body scanner to the vertical axis on basis of the depth map will be calculated.

At a further advantageous step, the optical indicator will be guided by controlling the position indicator projector. The position indicator projector is advantageously connected to the control unit. The control unit can control the position indicator projector in such a way that the position indicator projector will project the optical indicator at the optimal scanning position, which is arranged at the optimal scanning distance from the 3D body scanner and in particular in the middle in front of the 3D body scanner.

At a further advantageous step, the person to be scanned will be guided to the optical indicator by optical and/or audible signs. The user or the person to be scanned will be guided to the optimum position in front of the scanner for scanning and this position is automatic adapted to the body height of the user and how the scanner is installed in a room.

For guiding the user or person to be scanned, the 3D body scanner may comprise a direct communication interface, in particular a speaker, a buzzer, LED indicators as, for example, arrows and/or a display. Signs to guide the person to be scanned to the optimal scanning position can also be projected by the position indicator projector.

The method for interactive guiding the person to the optimum scanning position follows according at least one of the following steps. The user approaches the FOV of the 3D body scanner and the 3D body scanner switches, either user initiated or automatically from standby to active scanning mode. Then 3D body scanner determines the actual position of the user and starts guiding the user interactively by directions/advices to the optimum scanning position. The optimum scanning position is arranged at the optimum scanning distance, in particular furthermore in the middle, in front of the 3D body scanner. The advantage of this solution is that,by intuitively accepting to follow the directions/advices of the 3D body scanner to change the position of the body, no special user action is required. The interactive guiding of the user to the optimum scanning position is the following iterative process:
The 3D body scanner determines the geometrical relations (for example, but not limited to, distances, angles of floor, walls, objects, obstacles, user) in front of it and/or the geometrical installation of the 3D body scanner by its sensing components. This means 3D body scanner records, for example, depth map(s), which is in principle a 2D matrix of distance values, with its depth sensor(s) and/or RGB or black-white image(s) by its camera(s) of the room in front of it and optionally, if the user is already sufficiently visible, the body height of the user. Additionally or alternatively, the 3D body scanner may measure location data of, for example, but not limited to, objects or persons by Lidar or microwave radar.

The 3D body scanner calculates by geometrical equations from the geometrical relations and/or location data, the optimum scanning position of the user on the floor. This is done, for example, but not limited to, analyzing recorded depth maps, black-white, RGB or IR images and/or analyzing the location data.

The 3D body scanner determines the actual position of the user on the floor. This is done, for example, but not limited to, analyzing recorded depth maps, black-white, RGB or IR images and/or analyzing the location data.

The 3D body scanner calculates the length and direction of a difference vector between the actual position of the user and the optimum scanning position on the floor. This is done, for example, by its computation unit or within the computation unit of external device(s) or by cloud based processing.

The 3D body scanner checks if the actual position of the user is within a given circle of sufficient accuracy close to the optimum scanning position. If yes, the guiding process is ended.

If no, the 3D body scanner gives the user in an advantageous way a direction/advice how to move to the optimum scanning position. The direction/advice to the user may contain in advantageous way, for example, but not limited to, directional and/or distance information in absolute or relative scale. The direction/advice can be given, for example, either fix related to the main axis of view of the 3D body scanner or adaptive related to the direction of view of the user. The directions/advices to the user can be transmitted in an advantageous way, directly by the direct communication interfaces or indirectly by the indirect communication interfaces via connected external device(s).

The direction(s)/advice(s) given to the user may be of acoustical-, or optical or any other human noticeable nature. This can be for an acoustic way, in particular verbal (for example, voice) or non-verbal (for example, sound) and/or an optical way as, for example, written (static, running or animated text), static symbols or icons (for example, arrows or pictograms) and/or dynamic (live) symbols, icons or animations.

The 3D body scanner gives the user some time to move.

If the person is at the optimal scanning position the 3D body scanner gives a message that it is ready to scan or a given maximum number of iterations is reached and the 3D body scanner gives the message that a scan is not possible.

External device can be, for example, laptops, smartphones, tablets, and/or intelligent personal assistants.

Furthermore, a 3D body scanner for generating a 3D body model is proposed. The 3D body scanner is designed in such a way that the 3D body scanner can perform the method described in the previous and/or the following description. For example, the 3D body scanner may comprise a control unit, a position indicator projector and a sensor, in particular a depth sensor and/or a color camera, to measure the installation geometry of the 3D body scanner. The sensor, in particular a depth sensor and/or a color camera, can additionally or alternatively be used to scan the field of view to determine the body model of the user or the person.

In an embodiment of the 3D body scanner, the 3D body scanner and/or the position indicator projector comprises an actuator for tilting the position indicator projector, for tilting a mirror of the position indicator projector to deflect a light and/or laser ray and/or for adjusting a lens of the position indicator projector. With at least one of these features, the optical indicator can be guided to the optimal scanning distance.

In a further embodiment of the 3D body scanner, the position indicator projector comprises at least a light source, in particular LEDs, lasers, tungsten or halogen lamps. With at least one of these features, the optical indicator can be projected on the floor. Position indicator projector is designed in such a way that the position indicator projector can project, for example, a circle, a square and/or a star at the floor to indicate the optimal scanning distance for the person to be scanned. Furthermore, the position indicator projector is designed in such a way that the position indicator projector can project, for example, an arrow to show the person to be scanned the way to the optimal scanning distance or the optimal scanning position in front of the 3D body scanner.

In a further embodiment of the 3D body scanner, the 3D body scanner comprises an accelerometer for measuring the height of the 3D body scanner and/or an angle of the 3D body scanner according to a vertical axis.

The depth sensor is realized, for example, on basis of Stereo vision, assisted stereo vision, structured light, Time of flight, Lidar and/or Radar.

The optical and/or color camera is realized, for example, on basis of RGB, black-white and/or IR.

Additional advantages of the invention are described in the following exemplary embodiments. The drawings show in:
- **Figure 1**: two 3D body scanners at different heights,
- **Figure 2**: position indicator projector with tilted light or laser ray,
- **Figure 3**: position indicator projector with active light or laser ray and
- **Figure 4**: top view on the floor with the 3D body scanner, the user or person and the optimal scanning position.

The 3D body scanner can be in a small size with the intention to be placed somewhere on a furniture or hung on a wall in an only very roughly defined height and/or angular orientation. Usually, but not necessarily, turntables will be omitted. Nevertheless, for a best performance body scan, the user still needs to fill, as far as possible, but not exceed the field of view (FOV) in front of the 3D body scanner. The too large freedom in height and angular orientation of installation on furniture needs a situation dependent on compromising of the scanning distance of the user. Means the optimum scanning distance will change with the parameters of installation. It will be proposed a method to adaptively project an optical position indicator on the floor in front of the 3D body scanner in a distance which is optimum with respect to an installation geometry, in particular to installation height and placement angle of the 3D body scanner, for a given of a maximum body height of the user. This process is advantageously automatically run prior to any scan and works typically in three steps:
Determination of optimum scanning distance by measuring of installation geometry and geometric calculations.

Open- or closed-loop movement of light or laser ray angle using an electro-mechanical or electronic actuator.

Projecting of a convenient position indicator on the floor.

For simplicity of explanation in the next sections we state that the 3D body scanner emits a single light or laser ray and the optical indicator is assumed to be a single point at the optimum scanning position, even if it is a circle, ellipse, cross, star or any other convenient figure or pattern. Nevertheless, the 3D body scanner can also emit multiple light or laser rays.

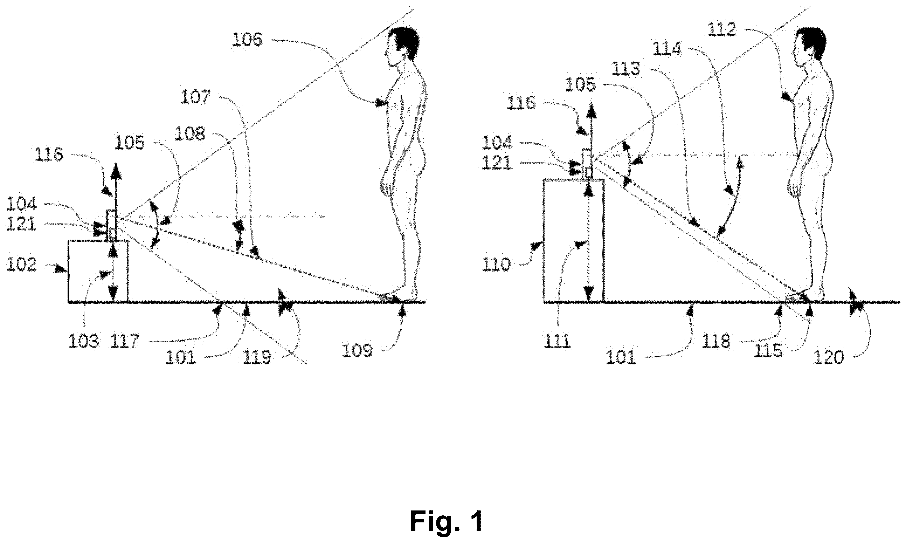

According to figure 1, two configurations of 3D body scanners 104 at different heights 103, 111 are shown. The optimum scanning distances are different when the 3D body scanners 104 are placed at different heights 103, 111 and the height axis 116 of 3D body scanner 104 is not fully vertical. The optimum scanning distances will be determined as given in the following examples:
The 3D body scanner 104 is placed, for example, on a low furniture 102 with the FOV of 3D body scanner 105 looking in the room. The configuration is shown on the left side of figure 1. The person to be scanned at low placed scanner 106 needs to be in a comparable far position. Limiting factor is that its head is still in the FOV of 3D body scanner 105. To determine the optimum scanning position of the person to be scanned 106, 3D body scanner 104 can do the following steps:
The 3D body scanner 104 records a depth map of the room in front of it and identifies the floor 101 as a plane in front.

The starting distance of floor plane 101 at low placed scanner 117 in the FOV 105.

The viewed angle 119 of the floor plane 101, and/or optionally if the 3D body scanner 104 has a built-in accelerometer, the angle of the height axis of 3D body scanner 116 related to vertical.

The a-priori knowledge of the FOV of 3D body scanner 105.

An assumption of the maximum height of the person to be scanned 112.

Therefrom, the 3D body scanner 104 calculates by geometrical relations the optimum scanning distance for the optical indicator on the floor at low placed scanner 107. When turning on the light or laser ray at low placed scanner 104 this projects an optical indicator 109 on the floor which shows where the user 116 has to step on.

Furthermore, the 3D body scanner 104 can also be placed, for example, on a high furniture 110 with its FOV 105 looking in the room. This configuration is shown on the right side of figure 1. The person 112 to be scanned at high placed scanner 104 can be in a comparable close position. This increases the resolution of the scanned person. Limiting factor is in this case that its feet are still in the FOV 105. To determine optimum scanning position of the person 112 to be scanned, 3D body scanner 104 calculates the angle of light or laser ray 114 to project an optical indicator 115 on the floor 101.

If the installation of the 3D body scanner 104 exceeds given limits and no appropriate scanning distance can be found, the 3D body scanner 104 may give in an advantageous way notice to the user 106, 112. The notice may be but not limited to an optical- or acoustical-, wired- or wireless- electronic one.

A feature of this invention is that the optical indicator can be moved to adapt on scanner installation geometry. There are at least, but not limited to, two advantageous ways to perform this movement:
- Electro-mechanical movement and/or
- Electronic movement

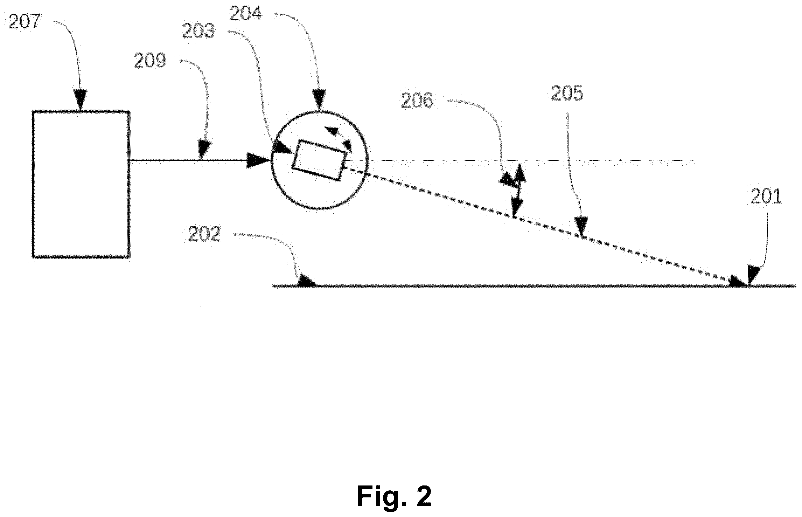

According to figure 2, the position indicator projector 203 is shown.

Features which are already described in the previous figure might not be explained again for the sake of simplicity. Furthermore, features may only be described in subsequent figures. Furthermore, for the sake of clarity, not all characteristics may be shown in the following figures. However, characteristics shown in one or more of the preceding figures may also be present in one or more of the following figures. Furthermore, for the sake of clarity, characteristics shown in one or more of the following figures may also be shown in one or more of the following figures. Nevertheless, features that are shown in one or more of the following figures may also be present in a previous figure. Furthermore, different reference signs can be used for the same features in the figures 1 - 4.

In the electro-mechanical solution, the control unit 207 of the 3D body scanner provides the angular position information as a digital or analog control signal 209 to an electro-mechanical actuator 204. This changes the angle of the position indicator projector 203 and therewith the angle 206 of the light or laser ray 205. The light or the laser ray 205 is projected onto the floor 202. The electro-mechanical actuator 204 for the motion of the ray 205 position indicator projector 203 may be realized:
In an advantageous analog electro-mechanical way as is, but not limited to, a rotatable or shiftable galvanometer coil, a piezo-electric actuator, or a servo motor.

In an advantageous digital way as is, but not limited to, a rotating or linear stepper motor.

In any other electro-mechanical way, which allows changing the position of the optical indicator 109, 115, 201 on the floor 101. The optical indicator 201 projected on the floor 202 may have in an advantageous way any shape which is best visible or otherwise convenient to the user. It may be, for example, but not limited to, a simple dot, a cross-hair, star, a filled or hollow square, circle or ellipse. Complicated indicator figures can be realized in an advantageous way by:
- using a beam shaped laser and/or
- using a lens-based position indicator projector 203

An also advantageous alternative electro-mechanical solution is using a fix mounted projector and a movable mirror tilted by an electro-mechanical actuator for changing the light or laser beam direction. This movable mirror can be a conventional mechanical solution, as, for example, mentioned above, or a solid-state circuit MEMS.

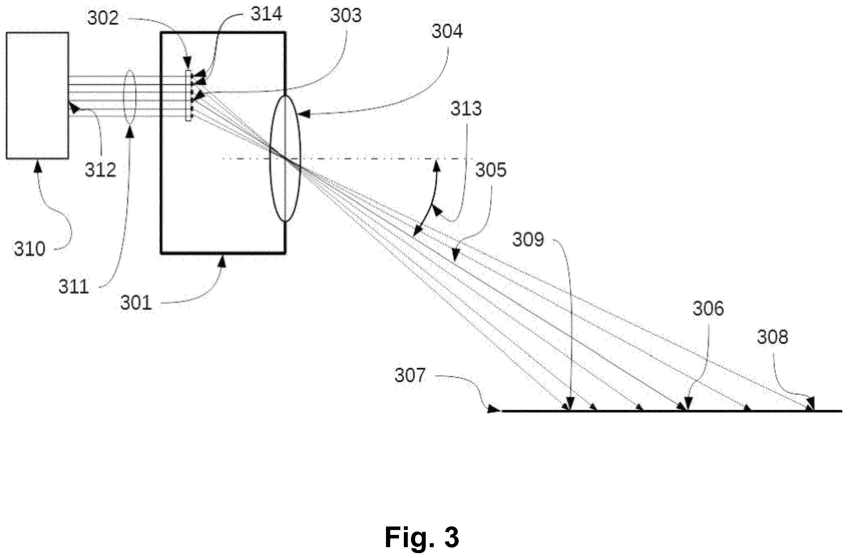

Figure 3 shows a further position indicator projector 301.

Features which are already described in one or more of the previous figures may not be explained again for the sake of simplicity. Furthermore, features may only be described in subsequent figures. Furthermore, for the sake of clarity, not all characteristics may be shown in the following figures. However, characteristics shown in one or more of the preceding figures may also be present in one or more of the following figures. Furthermore, for the sake of clarity, characteristics shown in one or more of the following figures may also be shown in one or more of the following figures. Nevertheless, features that are shown in one or more of the following figures may also be present in a previous figure. Furthermore, different reference signs can be used for the same features in the figures 1 - 4.

The invented electronic concept is based on projecting in an advantageous simple way the image of a light source 303 which is a part of an electronic actuator 302 by a projection lens 304 on the floor 307. This electronic concept is advantageous because it is noiseless and avoids costly and error-prone mechanical moving parts. The control unit 310 of the 3D body scanner provides the angular position information 311 as one or more control signal(s) 312 to an electronic actuator 302 which is a vector (line) of switchable light sources 314.

To change the tilt angle 313 of the projected light or laser ray 305 and therewith the position of the light indicator on the floor 307 only one or a limited number of such light sources 314 is activated by the control signal(s) 312 from a control unit 310. Dependent on the position of the active light source(s) 303 on the electronic actuator 302, the light or laser ray 305 can be moved between the closest light indicator position on floor 309 and the farest light indicator position on floor 308.

The granularity (distance steps) of the different discrete positions can be in an advantageous way selected by the number and distance of light sources in the vector (line) of light sources 302 and the angle of the electronic actuator 302 with respect to the optical axes of the projection lens 304. The light sources 314 may be in an advantageous way of any physical kind, as but not limited to, LEDs, lasers, tungsten or halogen lamps, or whatever light sources that can be manufactured in suitable format. The optical indicator 306 projected on the floor 307 may have in an advantageous way any shape which is best visible or otherwise convenient to the user. It may be, for example, but not limited to, a simple dot, a cross-hair, star, a filled or hollow square, circle or ellipse. Complicated static or dynamic symbols or figures can be realized in an advantageous way by:
- Composing the figures by multiple dots whereas the electronic actuator 302 can become a two-dimensional matrix of light sources or,
- Using for the actuator 302 a vector of beam shaped lasers.
- Using an LCD monitor instead of a simple vector or matrix of light sources, which allows to project stand-a still or dynamic "alive" images.
- Rotating the shape of the indicator could give in an advantageous way the user a hint about the targeted rotating speed at scanning.

The movement of the optical indicator to the appropriate scanning distance can be done, at least but not limited to, in two ways:
1. Open loop setting (steering) to save in an advantageous way hardware and software effort where the angle of light or laser ray 108, 114 for the targeted indicator position 109, 115 is simply calculated by the control unit 207, 310 of the scanner 104 and set without feedback by the electro-mechanical- actuator 204 or electronic- actuator 302.
2. Optional closed loop control to increase in an advantageous way accuracy and to combat manufacturing tolerances, where the calculated distance (position) of optical indicator on the floor 109, 115 is set as target and the real position of the indicator is moved by the electro-mechanical- 204 or electronic- 302 actuator in a closed loop using the difference between target and actual indicator position from a GB image captured by an optional RGB camera 121 of the scanner 104 as feedback information.

It is also advantageous that the concept can be used together with a turntable. In this case, the height of the turntable has to be provided to the scanner. Then this can correct its calculation by this height and project the indicator position the optimum location of the turntable. It is also possible that the scanner itself identifies the turntable and/or its height and automatically performs this correction. This identification can be done from depth map and/or RGB image.

The invention is not limited to the embodiments shown or described. Rather, any and all combinations of the individual features described, as shown in the figures or described in the description, and to the extent that a corresponding combination appears possible and sensible, are subject matters of the invention.

An advantageous aspect is that to adaptively project an optical position indicator 109, 115 on the floor 101 in front of the 3D body scanner 104 in a distance which is in an advantageous way optimum with respect to installation height 103, 111 and placement angle 116 of the 3D body scanner 104 for a given of a maximum body height of the user 106,112.

An advantageous aspect is that the determination of the optimum distance is in an advantageous way performed prior to every scan to ensure that intentional changes or changes by chance in the installation geometry of the 3D body scanner 104 are considered.

An advantageous aspect is that the 3D body scanner 104 in an advantageous way gives notice to the user which may be but not limited to, in optical- or acoustical- way, or a wired- or wireless- electronic way, if the geometrical relations of the installation are out of limit and/or no appropriate scanning distance can be found.

An advantageous aspect is that an electro-mechanical concept of optical indicator movement whereas a control unit 207 provides an angular position information as a digital or analog control signal 209 to an electro-mechanical actuator 204 which can change the angle of the indicator projector 203 and consequently the angle of the emitted light or laser ray 206 or directly angle of the emitted light or laser ray to change the distance of the optical position indicator 201.

An advantageous aspect is that the electro-mechanical actuator 204 is, in an advantageous analog electro-mechanical way, a, but not limited to, rotatable or shiftable galvanometer coil, a piezo-electric actuator, or a servo motor or in an advantageous digital way a, but not limited to, rotating or linear stepper motor, or any other electro-mechanical way.

An advantageous aspect is that the electro-mechanical solution with a fixed mounted projector and a tilt-able mirror, moved by an electro-mechanical actuator for optical indicator distance control.

An advantageous aspect is that the electronic concept of optical indicator movement is based on projecting in an advantageous way the image of a light source 303 by a projection lens 304 on the floor 307, and to change the angle of the projected light or laser ray 313 noiseless, without any mechanically moving parts by using an electronic actuator 302, which is vector (line) of consecutive light sources 314, activated by control signal(s) 312, whereas only one, or a limited number of the light sources, is made actively illuminating 303 and dependent on the position of the active light source(s) 303, the projected optical position indicator 306 is moved in distance on the floor 307.

An advantageous aspect is that the optical indicator projected on the floor may have in an advantageous way any shape which is best visible or otherwise convenient to the user as is, for example, but not limited to, a simple dot, a cross-hair, a filled or hollow circle or ellipse.

An advantageous aspect is that the optical indicator projected on the floor 307 may be in an advantageous way a standstill, alive or rotating image, which is electronic shiftable, displayed by an LCD screen, which is acting as electronic actuator 302.

An advantageous aspect is that the light sources may be in an advantageous way of any physical kind, as but not limited to, LED's, lasers tungsten or halogen lamps, or whatever light sources that can be manufactured in suitable format.

An advantageous aspect is that to save hardware and software effort is to use open loop setting (steering), where in advantageous way, the angle of light or laser ray 105, 206, 313 for the targeted indicator position is simply calculated by the control unit 207, 310 of the scanner and set without feedback by the electro-mechanical- 204 or electronic-actuator 302.

An advantageous aspect is that to increase, in an advantageous way, the accuracy of positioning and to combat manufacturing tolerances by using closed loop control, where the calculated distance (position) of optical indicator 109, 115, 201, 306 on the floor 101, 202, 307 is set as target and the real position of the indicator is moved by the electro-mechanical- 204 or electronic- 302 actuator and a closed loop using the difference between target and actual indicator position from a RGB image captured by an optional RGB camera 121 of the scanner 104 as feedback information for the control unit 207, 310 of the scanner 104.

Next generation of 3D body scanners will be in a small size with the intention to be placed somewhere on a furniture or hung on a wall in an only very roughly defined height and angular orientation. For a best performance body scan, the user needs to fill, but not exceed the Field Of View (FOV) in front of the 3D body scanner, whereas the optimum scanning distance will change with the parameters of installation. We invented a method to project an optical position indicator on the floor in front of the 3D body scanner in a distance which is optimum with respect to installation height and placement angle of the 3D body scanner for a given maximum body height of the user.

As can be seen, the features of the different figures can have different reference signs. For example, the floor has the reference sign 101 in figure 1, the reference sign 202 in figure 2 and the reference sign 307 in figure 3. Nevertheless, the feature may be the same. The same may hold for the other features.

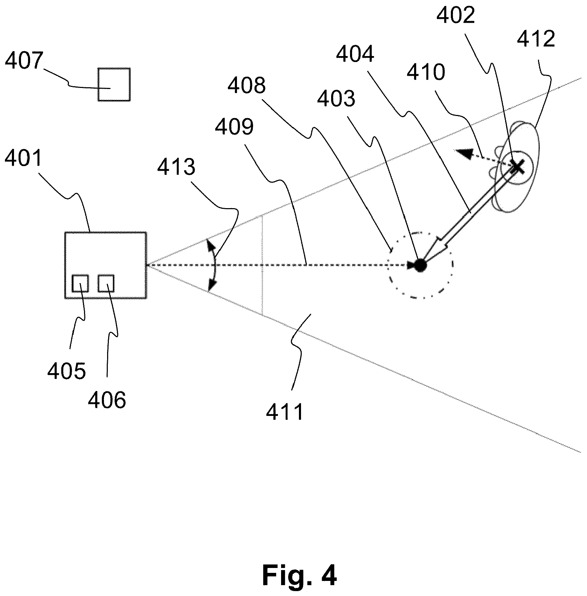

Figure 4 shows a top view of the 3D body scanner and the floor, the user or person and the optimal scanning position. Features which are already described in one or more of the previous figures may not be explained again for the sake of simplicity. Furthermore, features may only be described in subsequent figures. Furthermore, for the sake of clarity, not all characteristics may be shown in the following figures. However, characteristics shown in one or more of the preceding figures may also be present in one or more of the following figures. Furthermore, for the sake of clarity, characteristics shown in one or more of the following figures may also be shown in one or more of the following figures. Nevertheless, features that are shown in one or more of the following figures may also be present in a previous figure. Furthermore, different reference signs can be used for the same features in the figures 1 - 4.

The user approaches the field of view 413 of the 3D body scanner 401 and the 3D body scanner 401 switches, either user initiated or automatically from standby- to active scanning mode. Then 3D body scanner 401 determines the actual position 402 of the user 412 and starts guiding the user 412 interactively by directions/advices to the optimum scanning position 403. The advantage here is that, by intuitively accepting to follow the directions/advices of the 3D body scanner 401 to change the position of the body, no special user action is required. The interactive guiding of the user to the optimum scanning position 403 is the following, in particular iterative, process:
The 3D body scanner 401 determines the geometrical relations (for example, the distances, angles of floor, walls, objects, obstacles, user) in front of it by its sensing components. This means, 3D body scanner 401 records, for example, depth map(s), which is in principle a 2D matrix of distance values, with its depth sensor(s) and/or RGB or black-white image(s) by its camera(s) of the room in front of it and optionally, if the user is already sufficiently visible, the body height of the user. Additionally or alternatively, the 3D body scanner 401 may measure location data of, for example, but not limited to, objects or persons by Lidar or microwave radar.

The 3D body scanner 401 calculates by geometrical equations from the geometrical relations and / or location data, the optimum scanning position 403 of the user on the floor 411. This is done, for example, but not limited to, analyzing recorded depth maps, black-white, RGB or IR images and/or analyzing the location data.

The 3D body scanner 401 determines the actual position of the user 402 on the floor 411. This is done, for example, but not limited to, analyzing recorded depth maps, black-white, RGB or IR images and/or analyzing the location data.

The 3D body scanner 401 calculates the length and direction of the difference vector 404 between the actual position of the user 402 and the optimum scanning position 403 on the floor 411.

This is done, for example, by its computation unit or within the computation unit of external device(s) 407 or by cloud based processing. The computation can also be done by the control unit 310.

The 3D body scanner 401 checks if the actual position of the user 402 is within a given circle of sufficient accuracy 408 close to the optimum scanning position 403. If yes, the guiding process is ended.

If no, the 3D body scanner 401 gives the user in an advantageous way a simple direction/advice how to move to the optimum scanning position 403. The direction/advice to the user 412 may contain in advantageous way, for example, but not limited to, directional and/or distance information in absolute or relative scale. The direction/advice can be given, for example, either fix related to the main axis of view 409 of the 3D body scanner 401 or adaptive related to the direction of view of the user 410. The directions/advices to the user 412 can be transmitted in an advantageous way, directly by the direct communication interfaces 405 or indirectly by the indirect communication interfaces 406 via a connected external device(s) 407.

Here, the dotted line in figure 4 can also represent the optimal scanning distance 409 between the 3D body scanner 401 and the optimal scanning position 403.

The direction(s)/advice(s) given to the user may be of acoustical-, or optical or any other human noticeable nature. This can be, for example, an acoustic way like verbal (for example, voice) and/or non-verbal (for example, sound) and/or an optical way like written (static, running or animated text), static symbols or icons (for example, arrows or pictograms), dynamic (live) symbols, icons or animations.

The 3D body scanner 401 gives the user 412 some time to move.

These steps 1 to 7 of this interactive procedure are run in a loop until:
i. The user is found at step 5 to be within the circle of sufficient accuracy 408 and the 3D body scanner 401 gives the message that it is ready to scan or;
ii. The given maximum number of iterations is reached and the 3D body scanner 401 gives the message that a scan is not possible.

External device 407 can be, for example, but not limited to, a laptop, a smartphone, tablet and/or intelligent personal assistants.

An advantageous feature is that the user 412 is interactively guided to a scanning position in front of the 3D body scanner 401.

An advantageous feature is that the interaction between 3D body scanner 401 and user 412 is based on commands/directions given by the 3D body scanner 401 to the user 412 and the following reaction which is in the sense of the invention a change of body position which is noticed by the 3D body scanner 401 to create the following command/direction.

An advantageous feature is that the position in front of the 3D body scanner 401 as far as possible optimally uses the field of view 413 of the 3D body scanner 401 with respect to installation height 103, 111 (see fig. 1) and placement angle of the 3D body scanner 401 and the body height of the user 412 and potential present obstacles.

An advantageous feature is that by intuitively accepting to follow the directions/advices of the3D body scanner 401 to change the position of the body, no special user action is required.

An advantageous feature is that there is no need for the user 412 to measure the distance and direction from the optimum scanning position 403 to the 3D body scanner 401 and / or to place a permanent or temporary marker for orientation on the floor.

An advantageous feature is that interactive guiding of the user 412 to the optimal scanning position 403 while the 3D body scanner 401 determines the geometry of installation and/or measures location data and then calculates by geometrical equations from the installation geometry and object locations, the optimum scanning position 403 and the actual position 402 of the user 412 on the floor 411 from these the 3D body scanner 411 calculates the length and direction of the difference vector 404 between the actual position 402 of the user 412 and the optimum scanning position 403 and checks if the actual position of the user 402 within a given circle of sufficient accuracy 408 close to the optimum scanning position 203 which ends the guiding process and the 3D body scanner 401 gives the message that it is ready to scan or alternatively, the 3D body scanner 401 gives the user a direction/advice how to move to the optimum scanning position 403 and then the 3D body scanner 401 gives the user 412 some time to move and finally restarts this process until the given maximum number of iterations is reached.

An advantageous feature is that the 3D body scanner 401 determines geometry of installation and the location data of objects as, for example, but not limited to, walls, floor, persons or obstacles by sensing components.

An advantageous feature is that for determination of the installation geometry, and optionally, if the user is already sufficiently visible, the body height of the user, the 3D body scanner 401 records, for example, but not limited to, depth map(s), with its depth sensor(s) and/or RGB or black-white image(s) by its camera(s) of the room in front of the 3D body scanner 401.

An advantageous feature is that for determination of the installation geometry, especially objects in front of the 3D body scanner 401, the 3D body scanner 401 measures in an advantageous way, location data as, for example, distance and direction of objects, as, for example, but not limited to, obstacles or persons or the user 412 by Lidar or microwave radar.

An advantageous feature is that the 3D body scanner 401 determines the optimum scanning position 403 and the actual position 402 of the user 412 and, if the user is already visible sufficiently, the body height of the user, by analyzing the installation geometry and/or, if available, location data.

An advantageous feature is that the interactive guiding is an iterative process terminated by the user reaching the circle of required accuracy 408 around the optimum scanning position 403 and/or that the given maximum number of iterations is reached.

An advantageous feature is that the direction/advice to the user 412 may be, for example, but not limited to, of acoustical-, or optical or any other human noticeable nature.

An advantageous feature is that the calculations for determining the optimum scanning position 403 and the actual position of the user 412 are, for example, but not limited to, done by computation unit of the 3D body scanner 401, or within the computation unit of external device(s) 407 or somewhere in the cloud.

An advantageous feature is that the difference, shown as difference vector 401 between the optimum scanning position 403 and the actual position 402 of the user 412 (distance and/or direction) is used for guiding the user 412.

An advantageous feature is that the direction/advice to the user 412 may contain in advantageous way, for example, but not limited to, directional and/or distance information in absolute or relative scale and direction/advice can be given, for example, either fix related to the main axis of view 409 of the 3D body scanner 401 or adaptive related to the direction of view of the user 410.

An advantageous feature is that the directions/advices to the user 412 can be transmitted in an advantageous way, directly by the direct communication interfaces 405 and/or indirectly by the indirect communication interfaces 406 via a connected external device(s) 407.

An advantageous feature is that directions/advices to the user 412 can be provided, for example, but not limited to, in acoustic way as verbal (for example, natural or synthetic voice), non-verbal (for example, sound) and/or in an optical way as, for example, written (static, running or animated text), static symbols or icons (for example, arrows or pictograms), dynamic (live) symbols, icons or animations and/or any other human noticeable way.

### List of References

101: Floor
102: Low furniture
103: Height of low furniture
104: 3D body scanner (with control unit and optional accelerometer)
105: FOV of 3D body scanner
106: Person to be scanned at low placed scanner
107: Light or laser ray at low placed scanner
108: Angle of light or laser ray at low placed scanner
109: Optical indicator on floor at low placed scanner
110: High furniture
111: Height of high furniture
112: Person to be scanned at high placed scanner
113: Light or laser ray at low placed scanner
114: Angle of light or laser ray at high placed scanner
115: Optical indicator on floor at high placed scanner
116: Height axis of 3D body scanner
117: Starting distance of floor plane at low placed scanner
118: Starting distance of floor plane at high placed scanner
119: Viewed angle of floor plane at low placed scanner
120: Viewed angle of floor plane at high placed scanner
121: Optional RGB camera
201: Position indicator
202: Floor
203: Position indicator projector
204: Actuator
205: Light or laser ray
206: Tilt angle of light or laser ray
207: Control unit
209. Control signal for actuator
301: Position indicator projector
302: Electronic actuator (vector or line of light sources)
303: Active light source
304: Projection lens
305: Active light or laser ray
306: Optical indicator on floor
307: Floor
308: Farest light indicator on floor
309: Closest light indicator on floor
310: Control unit
311: Angular (position) information
312: Control signals
313: Tilt angle of light or laser ray
314: Light sources
401: 3D body scanner
402: actual position of the user
403: optimal scanning position
404: difference vector
405: direct communication interface
406: indirect communication interface
407: external device
408: circle of sufficient position accuracy
409: main axis of view / optimal scanning distance
410: direction of view of the user
411: floor
412: user
413: field of view of the 3D body scanner

## Claims

1. Method for operating a 3D body scanner (104, 401) for generating a 3D body model with the following steps:
calculating an optimal scanning distance between a person (106, 112, 412) to be scanned and the 3D body scanner (104, 401) by a control unit (207, 310) and
projecting an optical indicator (109, 201, 306) for the person (106, 112, 412) to be scanned on a floor (101, 202, 307, 411) in front of the 3D body scanner (104, 401) at the optimal scanning distance from the 3D body scanner (104, 401) by a position indicator projector (203, 301),
**characterized by** the step
of measuring an installation geometry of the 3D body scanner (104, 401) and calculating the optimal scanning distance between the person (106, 112, 412) to be scanned and the 3D body scanner (104, 401) by the control unit (207, 310) on basis of at least the installation geometry.

2. Method according to the previous claim,
wherein for the installation geometry an installation height of the 3D body scanner (104, 401) above the floor (101, 202, 307, 411) in front of the 3D body scanner (104, 401) is measured and/or
wherein for the installation geometry an installation angle of the 3D body scanner (104, 401) according to a vertical axis is measured.

3. Method according to one or more of the previous claims, wherein for calculating of the optimal scanning distance a field of view (FOV; 105, 413) of a depth sensor and/or a field of view (FOV; 105, 413) of a color camera, in particular a RGB camera, of the 3D body scanner (104, 401) are taken into account.

4. Method according to one or more of the previous claims with the further step:
scanning the field of view (FOV; 105, 413) by the depth sensor and/or by the color camera and generating a depth map by the control unit on basis of the data of the depth sensor and/or of the color camera.

5. Method according to one or more of the previous claims with the further step:
identifying the floor (101, 202, 307, 411) in front of the 3D body scanner (104, 401) on basis of the depth map.

6. Method according to one or more of the previous claims, wherein a body height of the person (106, 112, 412) will be determined on basis of the depth map.

7. Method according to one or more of the previous claims with the further step:
calculating the height of the 3D body scanner (104, 401) above the floor (101, 202, 307, 411) and/or the angle of the 3D body scanner (104, 401) to the vertical axis on basis of the depth map.

8. Method according to one or more of the previous claims with the further step:
moving the optical indicator (109, 201, 306) by controlling the position indicator projector (203, 301).

9. Method according to one or more of the previous claims with the further step:
moving the optical indicator (109, 201, 306) by controlling a two-dimensional matrix of light sources (314), in particular a vector line of light sources (314), of an electronic actuator (302) of the position indicator projector (203, 301) according to the optimal scanning position.

10. Method according to one or more of the previous claims with the further step:
guiding the person (106, 112, 412) to be scanned to the optical indicator (109, 201, 306) by optical and/or audible signs.

11. 3D body scanner (104, 401) for generating a 3D body model comprising a control unit for calculating an optimal scanning distance between a person (106, 112, 412) to be scanned and the 3D body scanner (104, 401) and a position indicator projector for projecting an optical indicator (109, 201, 306) for the person (106, 112, 412) to be scanned on a floor (101, 202, 307, 411) in front of the 3D body scanner (104, 401) at the optimal scanning distance from the 3D body scanner (104, 401), **characterized in**
**that** the 3D body scanner (104, 401) comprises a sensor to measure the installation geometry of the 3D body scanner (104, 401) and wherein the 3D body scanner (104, 401) is designed to perform the method for operating the 3D body scanner (104, 401) for generating the 3D body model according to one or more of the previous claims.

12. 3D body scanner (104, 401) according to the previous claim, wherein the 3D body scanner (104, 401) and/or the position indicator projector (203, 301) comprises an actuator for tilting the position indicator projector (203, 301), for tilting a mirror of the position indicator projector (203, 301) to deflect a light and/or laser ray and/or for adjusting a lens of the position indicator projector (203, 301).

13. 3D body scanner (104, 401) according to one or more of the previous claims, wherein the position indicator projector (203, 301) comprises at least a light source (314), in particular LEDs, lasers, tungsten or halogen lamps and/or
wherein the position indicator projector (203, 301) comprises an electronic actuator (302) with a two-dimensional matrix of light sources (314), in particular a vector line of light sources (314), and/or
wherein the position indicator projector (203, 301) comprises a lens (304) for projecting and/or focusing the light rays and/or the laser rays from the at least one light source (314) to the floor (101, 202, 307, 411).

14. 3D body scanner (104, 401) according to one or more of the previous claims, wherein the 3D body scanner (104, 401) comprises an accelerometer for measuring the height of the 3D body scanner (104, 401) and/or an angle of the 3D body scanner (104, 401) according to a vertical axis.

## Patentansprüche

1. Verfahren zum Betreiben eines 3D-Körperscanners (104, 401) zum Erzeugen eines 3D-Körpermodells mit den folgenden Schritten:
Berechnen eines optimalen Abtastabstands zwischen einer abzutastenden Person (106, 112, 412) und dem 3D-Körperscanner (104, 401) durch eine Steuereinheit (207, 310) und
Projizieren eines optischen Indikators (109, 201, 306) für die abzutastende Person (106, 112, 412) auf einem Boden (101, 202, 307, 411) vor dem 3D-Körperscanner (104, 401) im optimalen Abtastabstand von dem 3D-Körperscanner (104, 401) durch einen Positionsanzeigeprojektor (203, 301), **gekennzeichnet durch** den Schritt
des Messens einer Aufbaugeometrie des 3D-Körperscanners (104, 401) und des Berechnens des optimalen Abtastabstands zwischen der abzutastenden Person (106, 112, 412) und dem 3D-Körperscanner (104, 401) durch die Steuereinheit (207, 310) auf Grundlage zumindest der Aufbaugeometrie.

2. Verfahren nach dem vorangehenden Anspruch,
wobei für die Aufbaugeometrie eine Aufbauhöhe des 3D-Körperscanners (104, 401) oberhalb des Bodens (101, 202, 307, 411) vor dem 3D-Körperscanner (104, 401) gemessen wird, und/oder
wobei für die Aufbaugeometrie ein Aufbauwinkel des 3D-Körperscanners (104, 401) gemäß einer vertikalen Achse gemessen wird.

3. Verfahren nach einem oder mehreren der vorangehenden Ansprüche, wobei zum Berechnen des optimalen Abtastabstands ein Sichtfeld (FOV; 105, 413) eines Tiefensensors und/oder ein Sichtfeld (FOV; 105, 413) einer Farbkamera, insbesondere einer RGB-Kamera, des 3D-Körperscanners (104, 401) berücksichtigt werden.

4. Verfahren nach einem oder mehreren der vorangehenden Ansprüche, mit dem weiteren Schritt:
Abtasten des Sichtfelds (FOV; 105, 413) mit dem Tiefensensor und/oder der Farbkamera und Erzeugens eines Tiefenabbilds durch die Steuereinheit auf Grundlage der Daten des Tiefensensors und/oder der Farbkamera.

5. Verfahren nach einem oder mehreren der vorangehenden Ansprüche, mit dem weiteren Schritt:
Erkennen des Bodens (101, 202, 307, 411) vor dem 3D-Körperscanner (104, 401) auf Grundlage des Tiefenabbilds.

6. Verfahren nach einem oder mehreren der vorangehenden Ansprüche, wobei eine Körperhöhe der Person (106, 112, 412) auf Grundlage des Tiefenabbilds bestimmt wird.

7. Verfahren nach einem oder mehreren der vorangehenden Ansprüche, mit dem weiteren Schritt:
Berechnen der Höhe des 3D-Körperscanners (104, 401) oberhalb des Bodens (101, 202, 307, 411) und/oder des Winkels des 3D-Körperscanners (104, 401) zu der vertikalen Achse auf Grundlage des Tiefenabbilds.

8. Verfahren nach einem oder mehreren der vorangehenden Ansprüche, mit dem weiteren Schritt:
Bewegen des optischen Indikators (109, 201, 306) durch ein Steuern des Positionsanzeigeprojektors (203, 301).

9. Verfahren nach einem oder mehreren der vorangehenden Ansprüche, mit dem weiteren Schritt:
Bewegen des optischen Indikators (109, 201, 306) durch ein Steuern einer zweidimensionalen Matrix von Lichtquellen (314), insbesondere einer Vektorzeile von Lichtquellen (314), eines elektronischen Stellglieds (302) des Positionsanzeigeprojektors (203, 301) gemäß der optimalen Abtastposition.

10. Verfahren nach einem oder mehreren der vorangehenden Ansprüche, mit dem weiteren Schritt:
Leiten der abzutastenden Person (106, 112, 412) zum optischen Indikator (109, 201, 306) durch optische und/oder akustische Zeichen.

11. 3D-Körperscanner (104, 401) zum Erzeugen eines 3D-Körpermodells, umfassend eine Steuereinheit zum Berechnen eines optimalen Abtastabstands zwischen einer abzutastenden Person (106, 112, 412) und dem 3D-Körperscanner (104, 401) und einen Positionsanzeigeprojektor zum Projizieren eines optischen Indikators (109, 201, 306) für die abzutastende Person (106, 112, 412) auf einen Boden (101, 202, 307, 411) vor dem 3D-Körperscanner (104, 401) im optimalen Abtastabstand von dem 3D-Körperscanner (104, 401),
**dadurch gekennzeichnet,**
**dass** der 3D-Körperscanner (104, 401) einen Sensor umfasst, um die Aufbaugeometrie des 3D-Körperscanners (104, 401) zu messen, und wobei der 3D-Körperscanner (104, 401) ausgelegt ist, das Verfahren zum Betreiben des 3D-Körperscanners (104, 401) zum Erzeugen des 3D-Körpermodells gemäß einem oder mehreren der vorangehenden Ansprüche durchzuführen.

12. 3D-Körperscanner (104, 401) nach dem vorangehenden Anspruch, wobei der 3D-Körperscanner (104, 401) und/oder der Positionsanzeigeprojektor (203, 301) ein Stellglied zum Neigen des Positionsanzeigeprojektors (203, 301), zum Neigen eines Spiegels des Positionsanzeigeprojektors (203, 301), um einen Licht- und/oder Laserstrahls abzulenken, und/oder zum Einstellen eines Objektivs des Positionsanzeigeprojektors (203, 301) umfassen.

13. 3D-Körperscanner (104, 401) nach einem oder mehreren der vorangehenden Ansprüche, wobei der Positionsanzeigeprojektor (203, 301) mindestens eine Lichtquelle (314), insbesondere LEDs, Laser, Wolfram- oder Halogenlampen, umfasst und/oder
wobei der Positionsanzeigeprojektor (203, 301) ein elektronisches Stellglied (302) mit einer zweidimensionalen Matrix von Lichtquellen (314), insbesondere einer Vektorzeile von Lichtquellen (314), umfasst, und/oder
wobei der Positionsanzeigeprojektor (203, 301) ein Objektiv (304) zum Projizieren und/oder Fokussieren der Lichtstrahlen und/oder der Laserstrahlen von der mindestens einen Lichtquelle (314) auf den Boden (101, 202, 307, 411) umfasst.

14. 3D-Körperscanner (104, 401) nach einem oder mehreren der vorangehenden Ansprüche, wobei der 3D-Körperscanner (104, 401) einen Beschleunigungsmesser zum Messen der Höhe des 3D-Körperscanners (104, 401) und/oder eines Winkels des 3D-Körperscanners (104, 401) gemäß einer vertikalen Achse umfasst.

## Revendications

1. Procédé pour faire fonctionner un scanner corporel 3D (104, 401) pour générer un modèle corporel 3D, comprenant les étapes suivantes :
calculer, par une unité de commande (207, 310), une distance de balayage optimale entre une personne (106, 112, 412) à balayer et le scanner corporel 3D (104, 401) et
projeter un indicateur optique (109, 201, 306) pour la personne (106, 112, 412) à balayer sur un sol (101, 202, 307, 411) devant le scanner corporel 3D (104, 401) à la distance de balayage optimale à partir du scanner corporel 3D (104, 401) par un projecteur d'indicateur de position (203, 301), **caractérisé par** l'étape consistant à
mesurer une géométrie d'installation du scanner corporel 3D (104, 401) et calculer, par l'unité de commande (207, 310), la distance de balayage optimale entre la personne (106, 112, 412) à balayer et le scanner corporel 3D (104, 401) sur la base d'au moins la géométrie d'installation.

2. Procédé selon la revendication précédente,
dans lequel, pour la géométrie d'installation, une hauteur d'installation du scanner corporel 3D (104, 401) au-dessus du sol (101, 202, 307, 411) devant le scanner corporel 3D (104, 401) est mesurée et/ou
dans lequel, pour la géométrie d'installation, un angle d'installation du scanner corporel 3D (104, 401) par rapport à un axe vertical est mesuré.

3. Procédé selon l'une quelconque ou plusieurs des revendications précédentes, dans lequel, pour le calcul de la distance de balayage optimale, un champ de vision (FOV ; 105, 413) d'un capteur de profondeur et/ou un champ de vision (FOV ; 105, 413) d'une caméra couleur, en particulier une caméra RVB, du scanner corporel 3D (104, 401) sont/est pris en compte.

4. Procédé selon l'une quelconque ou plusieurs des revendications précédentes, comprenant en outre l'étape consistant à :
balayer le champ de vision (FOV ; 105, 413) par le capteur de profondeur et/ou la caméra couleur et générer une carte de profondeur par l'unité de commande sur la base des données du capteur de profondeur et/ou de la caméra couleur.

5. Procédé selon l'une quelconque ou plusieurs des revendications précédentes, comprenant en outre l'étape consistant à :
identifier le sol (101, 202, 307, 411) devant le scanner corporel 3D (104, 401) sur la base de la carte de profondeur.

6. Procédé selon l'une quelconque ou plusieurs des revendications précédentes, dans lequel une taille du corps de la personne (106, 112, 412) est déterminée sur la base de la carte de profondeur.

7. Procédé selon l'une quelconque ou plusieurs des revendications précédentes, comprenant en outre l'étape consistant à :
calculer la hauteur du scanner corporel 3D (104, 401) au-dessus du sol (101, 202, 307, 411) et/ou l'angle du scanner corporel 3D (104, 401) par rapport à l'axe vertical sur la base de la carte de profondeur.

8. Procédé selon l'une quelconque ou plusieurs des revendications précédentes, comprenant en outre l'étape consistant à :
déplacer l'indicateur optique (109, 201, 306) en commandant le projecteur d'indicateur de position (203, 301).

9. Procédé selon l'une quelconque ou plusieurs des revendications précédentes, comprenant en outre l'étape consistant à :
déplacer l'indicateur optique (109, 201, 306) en commandant une matrice bidimensionnelle de sources lumineuses (314), en particulier une ligne vectorielle de sources lumineuses (314), d'un actionneur électronique (302) du projecteur d'indicateur de position (203, 301) en fonction de la position de balayage optimale.

10. Procédé selon l'une quelconque ou plusieurs des revendications précédentes, comprenant en outre l'étape consistant à :
guider la personne (106, 112, 412) à scanner vers l'indicateur optique (109, 201, 306) par des signes optiques et/ou audibles.

11. Scanner corporel 3D (104, 401) pour générer un modèle corporel 3D, comprenant une unité de commande pour calculer une distance de balayage optimale entre une personne (106, 112, 412) à balayer et le scanner corporel 3D (104, 401) et un projecteur d'indicateur de position pour projeter un indicateur optique (109, 201, 306) pour la personne (106, 112, 412) à balayer sur un sol (101, 202, 307, 411) devant le scanner corporel 3D (104, 401) à la distance de balayage optimale à partir du scanner corporel 3D (104, 401), **caractérisé en ce que**
le scanner corporel 3D (104, 401) comprend un capteur pour mesurer la géométrie d'installation du scanner corporel 3D (104, 401) et dans lequel le scanner corporel 3D (104, 401) est conçu pour exécuter le procédé pour utiliser le scanner corporel 3D (104, 401) pour générer le modèle corporel 3D selon l'une quelconque ou plusieurs des revendications précédentes.

12. Scanner corporel 3D (104, 401) selon la revendication précédente, dans lequel le scanner corporel 3D (104, 401) et/ou le projecteur d'indicateur de position (203, 301) comprennent/comprend un actionneur pour incliner le projecteur d'indicateur de position (203, 301), pour incliner un miroir du projecteur d'indicateur de position (203, 301) afin de dévier un rayon lumineux et/ou laser et/ou pour ajuster une lentille du projecteur d'indicateur de position (203, 301).

13. Scanner corporel 3D (104, 401) selon l'une quelconque ou plusieurs des revendications précédentes, dans lequel le projecteur d'indicateur de position (203, 301) comprend au moins une source lumineuse (314), en particulier des LEDs, des lasers, des lampes au tungstène ou aux halogènes et/ou
dans lequel le projecteur d'indicateur de position (203, 301) comprend un actionneur électronique (302) comportant une matrice bidimensionnelle de sources lumineuses (314), en particulier une ligne vectorielle de sources lumineuses (314), et/ou
le projecteur d'indicateur de position (203, 301) comprend une lentille (304) pour projeter et/ou focaliser vers le sol (101, 202, 307, 411) les rayons lumineux et/ou les rayons laser provenant de l'au moins une source lumineuse (314).

14. Scanner corporel 3D (104, 401) selon l'une quelconque ou plusieurs des revendications précédentes, le scanner corporel 3D (104, 401) comprenant un accéléromètre pour mesurer la hauteur du scanner corporel 3D (104, 401) et/ou un angle du scanner corporel 3D (104, 401) par rapport à un axe vertical.
